# EUROPEAN PATENT SPECIFICATION

(11) **EP 4 391 976 B1**
(45) Date of publication and mention of the grant of the patent: **30.04.2025**
(21) Application number: 22801628.3
(22) Date of filing: 12.10.2022
(51) Int. Cl.: A61F 2/72, A61F 2/78

(54) **PROSTHETIC ASSEMBLY HAVING AN ELECTRODE INTERFACE FOR RECORDING MUSCLE ACTIVITY**
PROTHETISCHE ANORDNUNG MIT EINER ELEKTRODENSCHNITTSTELLE ZUR AUFZEICHNUNG DER MUSKELAKTIVITÄT
ENSEMBLE PROTHÉTIQUE AYANT UNE INTERFACE D'ÉLECTRODE POUR ENREGISTRER L'ACTIVITÉ MUSCULAIRE

(30) Priority: 15.10.2021 US 202163256015 P
(43) Date of publication of application: 03.07.2024
(73) Proprietor: Össur Iceland EHF, 110 Reykjavik (IS)
(72) Inventor: SVERRISSON, Atli Orn, 110 Reykjavik (IS); BACHE SIGURDARDOTTIR, Jona Sigrun, 110 Reykjavik (IS)
(74) Representative: Winger
(86) International application number: PCT/US2022/046364
(87) International publication number: WO 2023/064323

(56) References cited:
- WO-A1-2018/026842
- WO-A1-2021/080799
- DE-A1- 102017 126 463
- DE-B3- 102011 015 502
- US-A1- 2018 042 509
- US-B2- 9 155 634

## Description

### FIELD OF THE DISCLOSURE

The present disclosure relates to prosthetic assemblies, specifically electrode interfaces or assemblies, systems, and methods combined with a prosthetic liner and socket in a prosthetic assembly for recording muscle activity.

### BACKGROUND

Prosthetic liners are arranged to fit a residual limb and serve as a soft interface between the residual limb and a rigid prosthetic socket. As no residual limb is the same, there are challenges in effectively and intimately fitting a liner during use. These challenges relate to comfort, fit, and function, and include durability, moisture control, residual limb volume fluctuations, temperature, and migration or pistoning of the liner during use.

A prosthetic liner typically has a tubular and conical shape, with a first or proximal end being open-ended and a second or distal end being closed-ended. These liners are typically made from a layer of air-impermeable elastomeric material and may include a reinforcement layer intermediate between the inner and outer surfaces of the body portion of the liner or externally thereof to provide resistance against the axial elongation of the elastomer constituting the liner body. Such reinforcement typically does not restrict radial distension or stretching of the liner body. A textile cover may be on the outer surface of the liner and adjacent or layered relative to the layer of elastomeric material.

Prosthetic liners formed from elastomeric materials have been described in prior patents, such as, for example, U.S. Pat. No. 4,923,474 issued May 8, 1990; U.S. Pat. No. 5,507,834 granted Apr. 16, 1996; U.S. Pat. No. 5,376,129 granted Dec. 27, 1994; and U.S. Pat. No. 6,485,776, granted Nov. 26, 2002. Elastomeric liners are used to cushion a post-operative stump or residual limb regarding a prosthesis installed over the residual limb and coupled to the socket by a locking element described in U.S. Pat. No. 5,376,129. The suspension of the prosthesis occurs due to the suction of the liner against the residual limb.

It is desirable in such liners that they conform closely with the residual limb, accommodate all surface contours and sub-surface bone elements of the residual limb and provide a comfortable cushion between the residual limb and the hard socket of the prosthesis to be fitted over the residual limb.

Muscles may generate electrical impulses known as electromyographic (EMG) signals, which may be detected and amplified. An electrode interface or assembly can extract myoelectric signals and communicate user intention. For example, the electrode interface may be combined with a prosthetic liner and socket to extract myoelectric signals from amputees' residual limb and communicate intended movements.

Prosthetic devices perform well during stereotypic and cyclical activities such as walking and stair climbing. Still, they have difficulty detecting the user's intent to move his leg in other scenarios, such as rapidly moving between activities, walking in confined spaces, and during many non-cyclical activities during daily life. A potential solution is the addition of EMG signals to detect the user's intended movement.

Electrical signals can be transmitted between the residual limb and the outside of the prosthetic liner and socket. The EMG signals may be measured from the skin of the residual limb to control the prosthesis's function. The electrode interface can be arranged to detect muscle contraction signals at predetermined locations on the residual limb. Such signals can be recorded through various activities to provide beneficial information to improve prosthetic device control, such as operating a powered prosthetic knee, ankle, or foot. Such output from the electrical signals can be used to determine balance, the change of terrain or activities, and move the prosthesis at will without ambulation and/or other movement.

No EMG-controlled lower limb prostheses are commercially available to patients in the market, and limited studies exist on the usability and benefit of EMG control for lower limb prostheses. The lack of EMG control is partly due to the difficulty in obtaining robust EMG signals for control from the residual limb of amputees due to the user's prosthetic interface, which traditionally consists of the soft prosthetic liner and the rigid socket.

There is a difficulty in effectively providing an interface that accommodates the different structures of the prosthetic liner and rigid socket, as well as the material thickness, to interface with the skin of the residual limb while permitting access from outside the prosthetic assembly. In addition, as the prosthetic liner has an elastomeric configuration and the socket is rigid, stresses are caused between these structures and exert loads on an electrode assembly that typically extends through the thickness of the prosthetic liner and the rigid socket. Document DE 10 2011 015502 B3 discloses an array of metallic electrodes through the liner, to accommodate configurations of contacts from different devices.

High pressure and movement within the socket can cause difficulty in obtaining useful signals and produce noise and artifacts in EMG. The risk of skin damage to the residual limb is also a concern and places strict requirements on the design of the EMG system. Traditionally, the EMG recording electrodes have rigid components or long wires that are difficult to place within the soft liner and rigid socket without compromising user comfort and socket suspension. Documents US 2018/0042509 A1 discloses integration of conductive materials in textile liner, along a limb for directing signals from to a processing board. A similar layout is shown in document US 9 155 634 B2, which discloses the preamble of claim 1, with a gel liner. On the other hand, document WO 2021/080799 discloses elongated portions extending over liner and socket to connect to an external device. Document DE 10 2017 126463 A1 also shows long conductive paths embedded in clothes such as a T-shirt, however there are also electrode assemblies including a metal screw and nut.

Because of the foregoing discussion, there is a need for an electrode interface that is useable in combination with a soft prosthetic liner and a rigid socket, and effectively transmits EMG signals for adapting the prosthetic assembly.

### SUMMARY

Because of the problems encountered in known EMG interface systems in prosthetic assemblies, an electrode interface allows for effective EMG recording from within an existing soft prosthetic liner through a rigid socket during daily activities without compromising user comfort and socket suspension.

According to an embodiment of the disclosure, a stretchable conductive textile is used as an EMG electrode and an electrical conduit to transfer an electrical potential from the skin's surface along with a residual limb interior of the liner to the exterior of the liner. An EMG dome electrode may be embedded in the socket to provide electrical contact with the conductive textile and relay EMG signals to an EMG amplifier.

According to an embodiment of the electrode, the stretchable conductive textile includes portions corresponding to the interior and exterior sides of the prosthetic liner, with connecting portions to the interior and exterior portions. The interior portion is adapted to contact the skin of a residual limb and extends along the interior surface of the liner. The exterior portion is arranged to extend along the exterior surface of the liner for contact with the EMG dome connected to the socket.

The socket of the prosthetic assembly may be configured with the dome electrodes to conduct an electrical potential from the muscle of the residual limb through the prosthetic liner to the socket. The dome electrodes coincide with the exterior portion of the electrode. The exterior portion may be configured and dimensioned to enable sufficient surface area to allow for the liner and socket misplacement due to pistoning, rotations, or residual limb volume changes during the prosthetic assembly's use. In addition, the dome electrodes may be electrically connected to the amplifier via shielded wires to reduce electrical noise that may be picked up on the analog channels.

The prosthetic liner is preferably adapted with the interior portion of the electrode covering a predetermined surface area of the interior surface of the prosthetic liner, with at least one connecting portion extending through the thickness of the prosthetic liner to the exterior portion located along the exterior surface of the liner. Both the interior and exterior portions are preferably flush with the interior and exterior surfaces of the prosthetic liner so as not to raise any pressure points along with the prosthetic liner, either between the liner and the skin of the residual limb or between the liner and the socket.

The electrode may define the first and second connecting portions in a preferred embodiment. The first connecting portion is located between the interior and exterior portions, and the second connecting portion extends past the interior portion on an opposite side of the first connecting portion. For example, the second connecting portion may fold underneath the exterior portion or extend along the exterior surface of the liner outside the exterior portion of the electrode.

The prosthetic liner may be provided with at least two electrodes arranged in a predetermined configuration to obtain at least two EMG signals. The interior surface of the liner may be defined by an elastomeric material, whereas a textile cover may define the exterior surface of the liner. However, such electrode may be adapted according to different configurations of the liner and material compositions forming the interior and exterior surfaces of the liner.

According to a method of using the electrode interface in the prosthetic assembly, a user dons the EMG liner by rolling it onto the residual limb and then steps into the socket with embedded dome electrodes. The EMG recording system will measure the muscle activity within the liner through the textile electrodes during daily activities and send EMG activity to the relevant prosthesis.

From the embodiments and methods described according to the disclosure, the electrode interface offers soft and 2-D stretchable electrodes to increase comfort while balancing the sufficiency of electrical signals. As the electrode may be formed from a stretchable conductive textile, the solution offers an effective means of mounting electrodes in a soft prosthetic liner without compromising the efficacy and comfort of the prosthetic liner. Such a solution offers EMG recording while wearing a soft liner and a prosthetic socket, improving suspension and possibly reducing motion artifacts.

While a lower limb prosthetic assembly with a rigid socket is used in an exemplary embodiment, the electrode interface may be used for both upper limb and lower limb prosthesis.

Given the flexibility in selecting the textile-based electrodes' sizing and configuration, it is possible to configure and dimension the portions to allow for misplacement of the electrode when donning the liner. Such configuration may involve making the exterior portion larger than the interior portion or otherwise dissimilar in shape to account for pistoning/rotation/volume changes/misplacement while donning the socket.

As an advantage to the textile-based electrode, the shortest electrical connection, via configuration and dimensioning of the connecting portions, from the skin to the electronics located outside the socket can reduce possible noise pickup.

The electrode interface is not limited to a particular type of prosthetic liner but can be adapted in prosthetic liners of different material compositions and suspension types, including sealing liners, pin-based liners, socket shape and type, and prosthetic systems such as vacuum prosthetic liner and socket systems. Indeed, off-the-shelf liners may be adapted with the electrode interface, resulting in minimal modification to account for a user's individual needs.

When a user with an EMG-controlled prosthetic device dons the prosthesis, the user cannot turn on the prosthetic device before donning the prosthesis. Otherwise, the prosthetic device will be controlled with unreliable and noisy EMG signals and cause damage to the device, user, or the environment. The same goes for when the user doffs the prosthesis; the user must first turn off the device before doffing.

A donning and doffing system, according to the disclosure, solves these problems by identifying when the user has donned the prosthesis, and therefore EMG signals can be used to control the prosthetic device. Unreliable and noisy EMG signals can also occur when the socket fit is too loose, and the donning and doffing system will help identify and possibly reduce unwanted prosthetic movements. The donning and doffing system will also help determine when the EMG recording system can hibernate and save battery power. If the prosthetic device only relies on EMG activity for control, then the prosthetic device can hibernate and save energy.

According to another aspect of the disclosure, residual muscle activity is believed to provide information on the intent of the user to move his leg. Thus, by recording EMG signals through various activities, beneficial information is provided to improve prosthetic device control. The lack of EMG control in lower extremity prostheses is in part due to the difficulty in obtaining robust EMG signals for control from the stump of amputees. High pressures and movement within the socket cause difficulty in obtaining useful signals and can produce noise and artifacts in EMG. Tracking EMG control signals reduces unwanted motion artifacts during use and creates additional control signals for bionic prosthetics. The tracking of the EMG control signal allows the user to voluntary control the prosthesis and provides the user's intent.

Current bionic prostheses rely mostly on mechanical sensors (IMU, load cells, etc.) to know the user's intent by capturing the user's movement as input into the prosthetic state machine, which ultimately creates the prosthetic movement. The embodiments described herein enable the user to provide the bionic prosthesis with an additional control signal to move the prosthesis according to the user's intent without needing a limb movement.

Specifically, the tracking system defines an EMG recording system with an IMU that measures EMG signals and inertial signals from the residual limb. The EMG Error Checker uses EMG values and the acceleration jerk magnitudes to identify and remove motion artifacts in the EMG signals.

These and other features, aspects, and advantages of the present disclosure will help better understand the following description, appended claims, and accompanying drawings.

### BRIEF DESCRIPTION OF THE DRAWINGS

Fig. 1 is an exemplary perspective view of a prosthetic assembly, including an embodiment of an electrode interface.
Fig. 2A is a plan view of an exemplary textile-based electrode.
Fig. 2B is a cross-sectional view of a liner having a variation of the textile-based electrode of Fig. 2A.
Fig. 2C is a plan view of the textile-based electrode of Fig. 2B.
Fig. 2D is a cross-sectional view of a liner having another variation of the textile-based electrode of Fig. 2A.
Fig. 2E is a plan view of the textile-based electrode of Fig. 2D.
Fig. 3 is a perspective view of detail III taken from Fig. 1, showing an exterior surface of the liner located against a prosthetic socket.
Fig. 4 is a perspective view of an interior surface of a liner in the embodiment of Fig. 1.
Fig. 5 is a schematic view of the electrode interface in the embodiment of Fig. 1.
Fig. 6 is a schematic view of a variation of the electrode interface in Fig. 5.
Fig. 7 is a schematic view of another variation of the electrode interface in Fig. 5.
Fig. 8 is a schematic view of a variation of an electrode interface in another prosthetic assembly.
Fig. 9 plots EMG data from a transtibial user donning a prosthetic assembly during a non-ambulating and weight-bearing situation.
Fig. 10 plots EMG data from a transtibial user during level ground.
Fig. 11A is a schematic view of an electrode interface for determining the status of donning and doffing of the liner in a connected or donned configuration.
Fig. 11B is a schematic view of the electrode interface of Fig. 11A in a doffed or disconnected configuration.
Fig. 11C is a schematic view of a variation of the electrode interface of Fig. 11A.
Fig. 12A plots EMG data of level ground walking with two voluntary contractions during the swing phase for an EMG control signal.
Fig. 12B plots the EMG data of Fig. 12A with a reduction of motion artifacts.
Fig. 13A plots EMG data of level ground walking with two voluntary contractions during the swing phase for an EMG artifact removal.
Fig. 13B plots the EMG data of Fig. 13A with a reduction of motion artifacts.

In the various figures, similar elements are provided with similar reference numbers. The drawing figures are not drawn to scale or proportion but instead are drawn to understand the components better and are not intended to be limiting in scope but rather provide exemplary illustrations.

### DETAILED DESCRIPTION OF VARIOUS EMBODIMENTS

A better understanding of different embodiments of the disclosure may be had from the following description read with the accompanying drawings in which reference characters refer to like elements.

While the disclosure is susceptible to various modifications and alternative constructions, certain illustrative embodiments are in the drawings described below. It should be understood, however, that there is no intention to limit the disclosure to the embodiments disclosed, but on the contrary, the intention covers all modifications, alternative constructions and combinations falling within the scope of the disclosure.

It will be understood that unless a term is defined in this disclosure to possess a described meaning, there is no intent to limit the meaning of such term, either expressly or indirectly, beyond its plain or ordinary meaning.

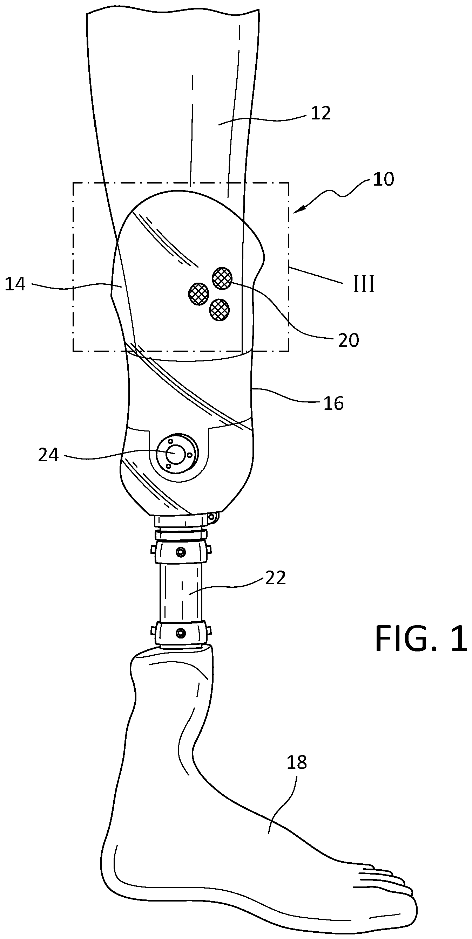

Fig. 1 is an exemplary embodiment of prosthetic assembly 10 for a transtibial prosthesis. The prosthetic assembly 10 includes a prosthetic liner 12 adapted to envelop a distal end of a residual limb. The prosthetic liner 12 is received by a rigid socket 14, including a release valve 24. As readily understood by the skilled artisan in prosthetics, an example of a method for making a socket is described in U.S. Pat. No. 7,438,843, granted Oct. 21, 2008. The prosthetic liner 12 includes a seal component 16, an example described in U.S. Pat. No. 7,025,793, and U.S. application publication 2007/0123998.

The prosthetic assembly 10 includes an electrode interface 20 that communicates from the interior of the prosthetic assembly or interior surface of the prosthetic liner 12 to the exterior of the prosthetic assembly 10 and exterior of the socket 14.

The socket 14 is connected to a prosthetic device, such as the depicted pylon 22. The pylon 22 is considered exemplary as the socket 14 may secure to other prosthetic devices such as a mechanical or powered knee in a transfemoral prosthetic assembly, or via the pylon to a prosthetic foot 18 and/or to a prosthetic foot 18 via a powered prosthetic ankle 24.

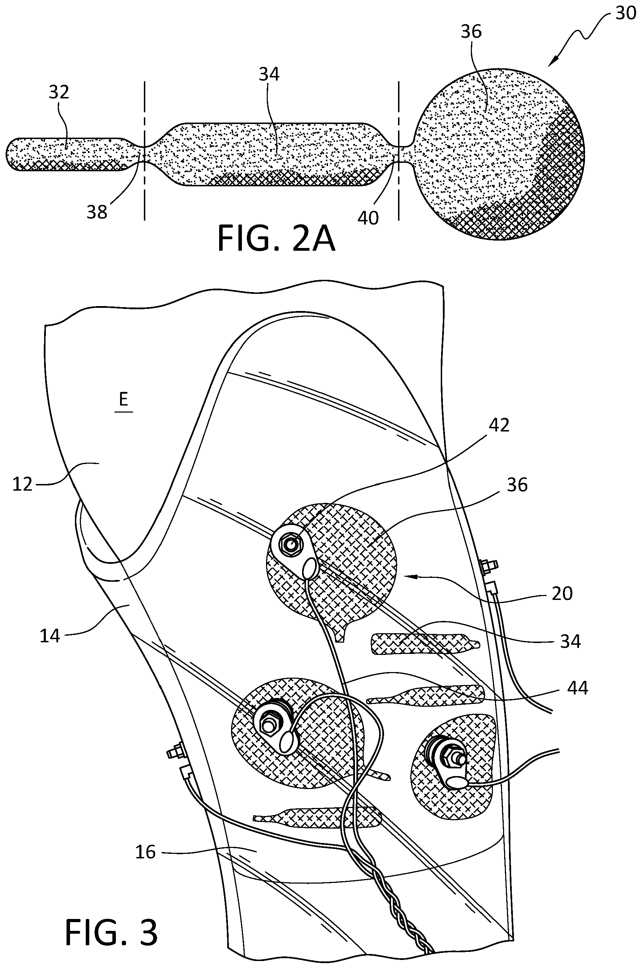

Fig. 2A shows an exemplary electrode 30 according to an embodiment of the disclosure. The electrode 30 may be a conductive textile-based electrode having flexibility and is capable of being applied to a prosthetic liner, as will be shown in subsequent embodiments. The textile-based electrode may be a silver-plated knitted fabric, for example, 78% Polyamide + 22% Elastomer and 99% pure silver plated. The textile-based electrode may be selected for bidirectional stretch with low surface resistivity and small thickness so as not to create any pressure points. An exemplary textile-based electrode is Shieldex Technik-tex P130+B, provided by Statex Produktions- und Vertriebs GmbH of Bremen, Germany.

The electrode 30 includes a first exterior portion 32, thereby adapted to the exterior surface or side of the prosthetic liner and arranged to be adjacent to an interior surface of the socket. The first exterior portion 32 is followed by a first connecting portion 38 connecting to an interior portion 34 adapted to the interior surface or side of the liner and arranged to be adjacent to the skin of a residual limb when the prosthetic liner is donned. A second connecting portion 40 links the interior portion 34 to a second exterior portion 36, adapted to the prosthetic liner's exterior surface and arranged adjacent to an interior surface of the socket.

The first and second exterior portions 32 and 36 may be configured and dimensioned according to the degree of contact desired with corresponding dome electrodes placed about the socket, as shown and discussed in connection with Fig. 3. For example, the first exterior portion 32 may serve a purpose, at least in part of securing the electrode 30 to a liner. In contrast, the second exterior portion 36, while also serving in part to secure the electrode to the liner, is configured and dimensioned larger with greater surface area to accommodate possible movement between the liner and socket. In the exemplary depiction, the second exterior portion 36 is round and has a considerably greater surface area than the first exterior portion 32. The large circular shape of the second exterior portion 36 on the outside of the liner allows for misplacement of the liner and socket due to pistoning, rotations, or limb volume changes.

The larger diameter or size of the second exterior portion 36 may be chosen to an ideal size of 2 to 4 cm, and more preferably about 3 cm to allow for misalignment, pistoning, and rotation in a transfemoral prosthetic liner. Alternatively, for a transtibial prosthetic liner, the second exterior portion 36 may be chosen to be of smaller size, such as between 1 to 3 cm, and more preferably about 2 cm, since there is less soft-tissue at a residual limb to cause misalignment. Therefore, the electrode may be adapted to different sizes depending on the prosthetic liner type and the size of an individual residual limb (larger versus smaller).

The interior portion 34 may possess an elongated configuration, thereby being longer than wider and configured and dimensioned to sufficiently and consistently enable effective and successful EMG signals to be obtained. The connecting portions may be sized and configured to a width or standard width of a prosthetic liner, so the interior portion and the exterior portions are flush with the interior and exterior surfaces, respectively, of the prosthetic liner without protruding or significantly protruding beyond the interior and exterior surfaces of the prosthetic liner to create pressure points.

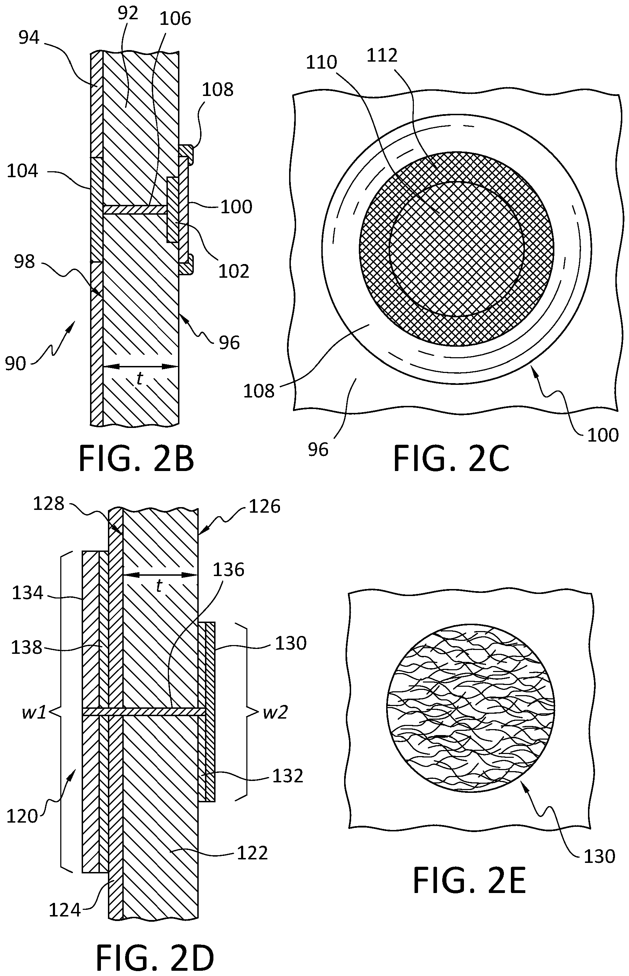

Figs. 2B and 2C illustrate a variation 90 of the textile-based electrode of Fig. 2A. A liner 92 having a thickness of t and a textile layer 94 extends along an outer side 98 of the liner 92. The liner 92 defines an inner side 96 along which a (an inner) conductive textile segment 100 extends. A textile segment 102 may extend below the inner side 96 and adjacent to the textile layer 94. A connective yarn 106 connects the conductive textile segment 100 to a (an outer) conductive textile segment 104 on the outer side 98. A silicone adhesive 108 extends about the conductive textile segment 100.

The silicone adhesive 108 (such as MEDx-4013 from NuSil Technology LLC of Carpinteria, CA) is heat cured to securely integrate the conductive textile segment 100 onto the inner side of the liner 96. The silicone adhesive 108 extends around edges 112 of the conductive textile segment 100, which may include a border 112, and exposes only the electrode (conductive textile) 110 where intended. The silicone adhesive 108 and the electrode 100 stretch with the liner and create a robust solution. The conductive yarn 106 is sewn in the textile segment 102 located beneath the electrode 100, and the yarn 106 is pulled through the liner and sewn into the conductive pad 104 along the textile layer 94 of the liner 92, which is then later glued to the textile layer. The textile segment 102 beneath the conductive textile segment 100 can be conductive or not. The conductive yarn 106, therefore electrically connects the interior side 96 and the exterior side 98 through a single point.

Figs. 2D and 2E illustrate another variation 130 of the textile-based electrode of Fig. 2A, specifically by securing a (an inner) conductive textile segment 130 to a liner 92 with a silicone adhesive sheet 130. The conductive textile segment 130 is securely fastened to the inner side 126 of the liner 122 using a primer. An adhesive sheet, such as a silicone sheet 132 having part name R1-2680-4 from NuSil Technology, on the conductive textile segment 130, and subsequently pressing them together. The conductive textile segment 130 may be cut to a shape, as shown by the circle, although other shapes are possible.

The conductive textile segment 130 adheres to the silicone sheet 132, and they are placed on the desired location on the inner side 126 of the liner 122, with suitable pressure and/or heat to accelerate curing of the silicone sheet 132 to the inner side 126 of the liner, which is preferably formed from a silicone material. A conductive thread 136 is threaded through the conductive textile segment 130 and through a thickness t of the liner 122 to the outer side 128 of the liner 122 to make an electrical connection between the (inner) conductive textile segment 130 to a (an outer) conductive textile segment 134.

The conductive textile segment 134 may adhere to a textile layer 124 extending along or defining the outer side 128 of the liner by an adhesive 138. As shown, the inner conductive textile segment 130 may be sized differently from the outer conductive textile segment 134, in part so the outer conductive textile segment 134 can have a width or surface area w1 sufficiently large to a corresponding electrode on a socket when the inner conductive textile segment 130 has a width w2 or surface area smaller due to a lower need for greater surface area.

Fig. 3 shows the liner 12 located within the socket 14, and the dome electrodes 42 in correspondence or generally in correspondence with at least the second exterior portions 36 to obtain signals from the interior of the liner against the skin of the residual limb. The dome electrodes 42 are electrically connected to an amplifier (not shown) via shielded wires 44 to reduce electrical noise that may be picked up on analog channels.

An example of dome electrodes may be remote electrodes manufactured by Össur hf of Reykjavik, Iceland, and found in its Prosthetic Solutions Catalog, Upper Extremity 2019 Catalog. These electrodes may be but are not limited to gold-plated domes for obtaining an improved EMG signal. The electrodes may operate at 50 Hz or 60 Hz and have 10 mm diameter electrode domes. Due to their size and configuration, the dome electrodes may offer improved comfort within the socket as they can closely follow the contours of a residual limb. An O-ring or gasket may be placed between the dome electrodes to maintain a vacuum in a socket between the prosthetic liner and the socket.

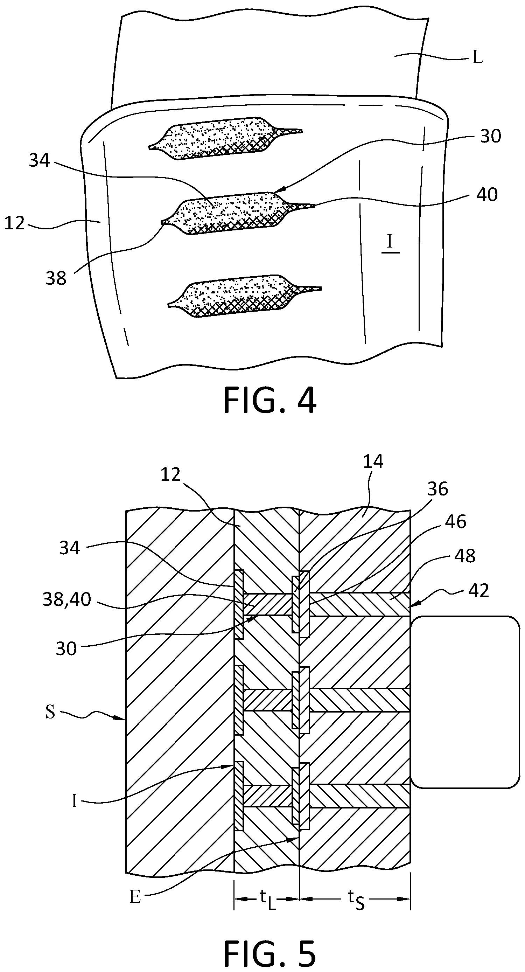

Fig. 4 illustrates that at least one pair of electrodes 30 for differential measurement must be located above or close to a designated muscle that is to be recorded from. A ground electrode may also be included and optimally, at least one electrode for each muscle and between the two differential electrodes. Exemplary muscles include the tibialis and gastric medialis for transtibial amputees and the quadriceps and hamstrings for transfemoral amputees.

As shown in Fig. 5, the dome electrodes 42 preferably extend through a thickness tₛ of the socket so that at least an interior portion 46 corresponds with the second exterior portion 36 of the electrode 30. An exterior portion 48 of the dome electrode 42 is arranged to communicate or transmit signals to electronics to gather and read the EMG signals obtained from the skin S of the residual limb.

Fig. 5 also exemplifies the electrode 30 as extending through a thickness t₁ of the liner 12, and the liner's interior surface IL directly adjacent to the residual limb's skin S, and the exterior surface EL directly adjacent to the interior surface Iₛ of the socket 14.

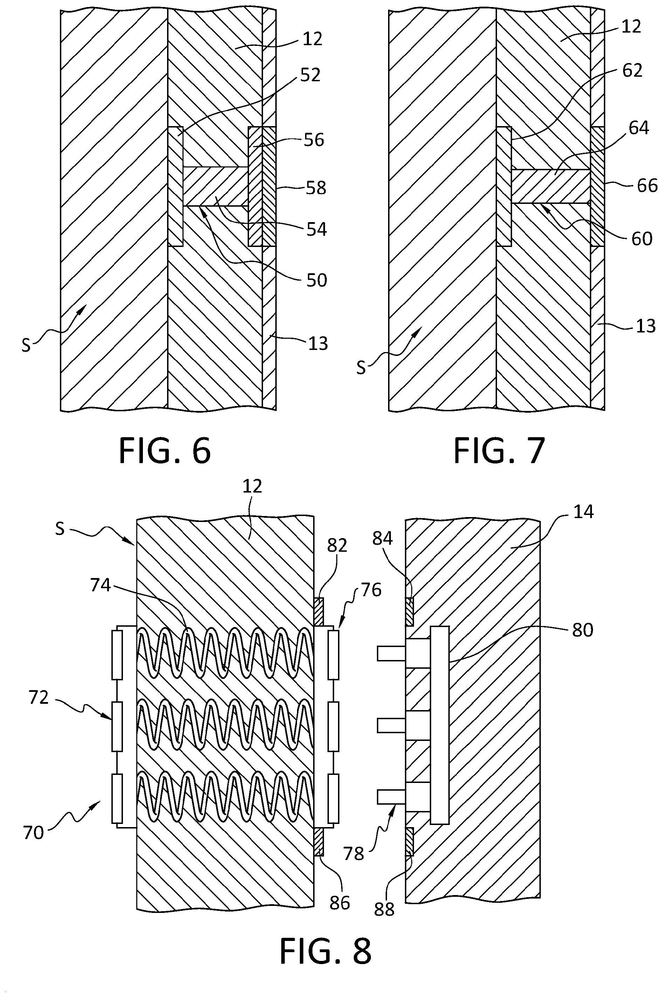

Fig. 6 shows a variation of an electrode interface 50. In this embodiment, the connecting portion 54 is a conductive rubber communicating the interior portion 52 to the exterior portion 56. The exterior portion 56 may comprise a conductive textile 58, which is generally flush with a textile cover 13 of the liner 12.

Fig. 7 illustrates another variation of an electrode interface 60. In this embodiment, the interior portion 62 is a conductive rubber, textile, gel or sponge. The connecting portion 64 is a conductive gel, which extends to the exterior portion 66, which may be a conductive textile or other conducting material extending along the exterior surface of the liner.

Fig. 8 depicts yet another variation of an electrode interface 70. The electrode interface 70, such as a printed circuit board (PCB), includes an internal array of electrodes 72 disposed along the interior surface of the liner 12. The electrode array 72 is connected to springs or conductive threads 74 that couple to an external array of electrodes 76 disposed along an exterior surface of the line. The socket 14 includes a pin array 78 adapted to correspond to the exterior electrode array 76. Various corresponding magnets 82, 84, 86, and 88 are located about the pin array 78 and the external electrode array 76 for lining them up to one another. A printed circuit board 80 communicates to the pin array 78 for obtaining a transmission of electrode signals.

Metal electrodes may be on the PCB, and the PCB is preferably flexible. A single PCB may be provided for all electrodes to measure one muscle or a designated muscle group.

In any of the foregoing electrode interface embodiments, they are arranged to be adapted to an off-the-shelf or custom liner, such that the liner is not manufactured with the electrode interface. Rather, the electrode interface is an optional component that can retrofit an already manufactured prosthetic liner, or may be integrated into a custom prosthetic liner or a prosthetic liner, as in U.S. patent application publication 2021/0137708, published on May 13, 2021.

According to variations of any of the embodiments above, the conductive textile electrode can be in various sizes and shapes for larger or smaller muscles. In a variation, the liner electrodes are integrated along the interior surface, and conductive tracks are provided, which extend proximally to a connector at a proximal or distal end of the liner. Alternatively or additionally, conductive tracks may extend distally to the liner's distal pin connector or a magnetic connector at the distal end of the prosthetic socket. Other alternatives include electrodes that are single-use or permanently fixed in the liner or comprise conductive ink printed onto the liner to create electrodes.

Various other configurations are possible in connecting electrodes along an interior surface of the liner, such as extending through punched holes through a thickness of the liner. Liner electrodes may be embedded in a sock or sweat sock and used in conjunction with the EMG liner to improve socket fit, either underneath or outside the liner.

As shown above, the electrodes must not necessarily be derived from a textile-based electrode, but various portions of the liner electrodes may be composed of conductive silicone. For example, the liner electrodes may be composed of a conductive silicone/rubber glued to the inside, conductive textile glued to the outside textile of the liner, and the conductive material in between can be rubber, gel, sponge, foam, or any soft or flexible or compressible material that is conductive or covered with a conductive material. Alternatively, the textile electrodes can be a flexible printed circuit board.

The liner may be adapted or prefabricated with a pocket or hole for the electrode to be plugged in. A signal conveyed can be from another sensor/actuator within the liner, and this solution makes it possible to send/receive signals and/or power to the liner from the socket. An alternative sensor could measure temperature, humidity, pressure, force, shear, magnetic flux, blood flow, oxygen content, inertial movements, etc.

An actuator may be embedded in the liner to provide sensory feedback, alerts or warnings, massaging, pressure changes, volume changes, etc. The liner electrodes are cast and/or cured simultaneously as the silicone liner. Any conductive track/textile that is not supposed to contact another item can be shielded to reduce signal noise. Likewise, the entire socket can be shielded to reduce signal noise. Conductive sockets can be covered with a nonconductive material to not be electrically short between dome electrodes or between conductive textile electrodes.

The dome electrodes can be spring-loaded to keep constant contact. The dome electrodes in the rigid socket can be any electrical connector (e.g., pogo pin) that makes an electrical connection via touch.

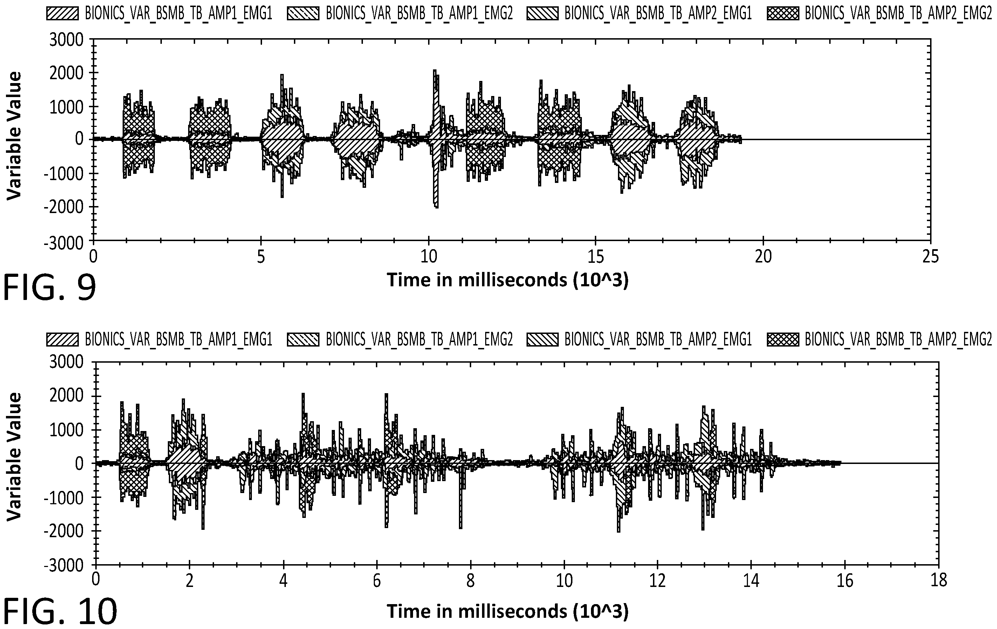

Fig. 9 shows an example of EMG data from a transtibial user during a non-ambulating and weight-bearing situation where the user dorsiflexes twice, then plantarflexes twice, and repeats.

Fig. 10 shows an example of EMG data from another transtibial user during level-ground walking with two contractions at the start, first dorsiflexing and then plantarflexing. Then during walking, the user dorsiflexes twice in the first walk and then plantarflexes twice in the next walk.

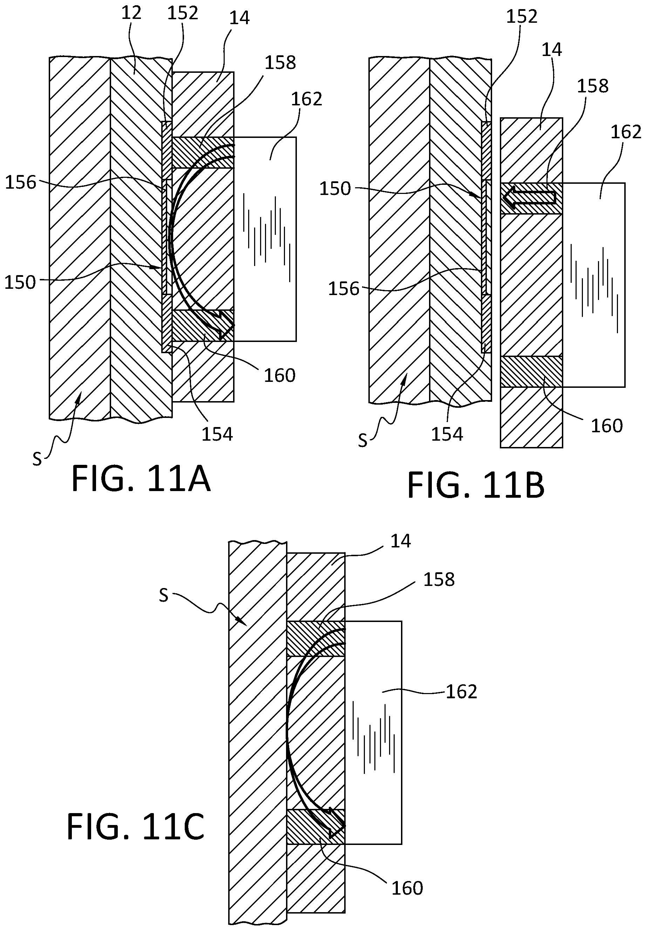

Figs. 11A-11C exemplify an EMG donning and doffing system 150, where two or more electrically conductive pads/locations on the exterior of the liner (textile side) are electrically connected and mate with electrically conductive elements in the socket when the prosthesis is donned. The electrically conductive elements in the socket are connected to an electronics board where conductivity is measured between the elements. If they connect all through the liner, then the user has donned the liner and socket; if they are not electrically connected, then the user has doffed the socket.

Two or more conductive pads, such as any of the embodiments above, are electrically connected on opposite sides of the liner with conductive textile strips or conductive yarns. The hard socket is modified with dome electrodes/pogo pins, located above or over the periphery of a shape of the conductive pads on the outside of the liner, allowing for misplacement of the liner and socket due to pistoning, rotation or stump volume changes. The dome electrodes/pogo pins are electrically connected to an electronic board that measures conductivity or resistance to determine if the socket is properly donned.

Accordingly, the donning and doffing system allows the EMG-controlled prosthesis to be donned/doffed while the prosthesis is turned on without causing unintentional prosthetic movement. The EMG-controlled prosthesis can hibernate to save energy when doffed, which is not possible today without the user actively turning off the prosthesis. The EMG-controlled prosthesis can resist making unintentional movement when socket fit is not proper, and the likelihood of a motion artifact is high. The system can provide an indication that the socket fit might not fit properly.

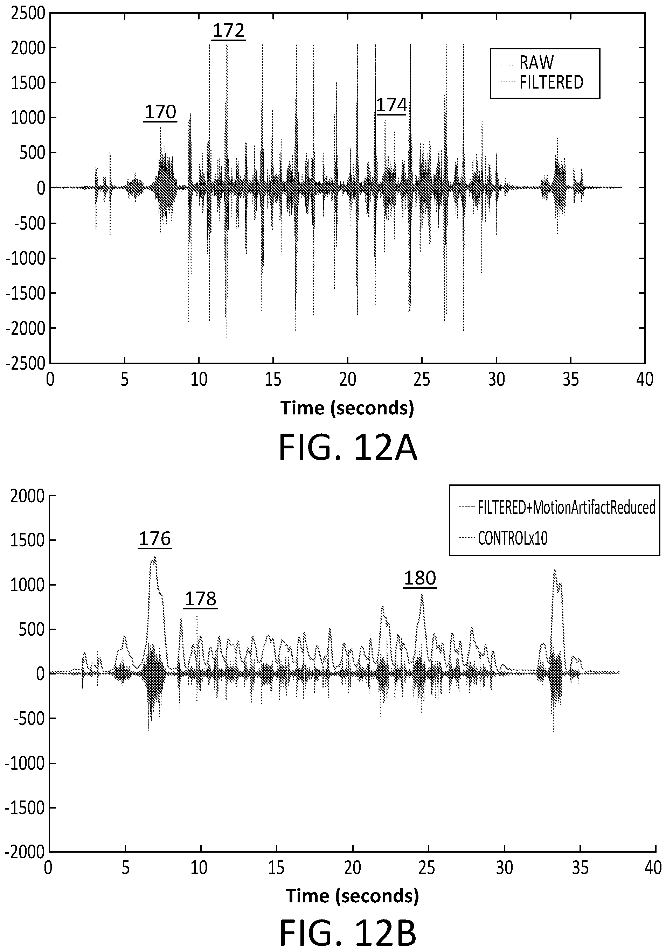

Figs. 11A and 11B exemplify an embodiment with the skin S of the user being against the inner side of the liner 12, which includes the donning and doffing system 150. The donning and doffing system 150 has first and second electrode pads 152, 154, connected by a conductive thread or another connector 156. The socket 14 has the dome electrodes or pogo pins 158, 160 connected to electronics 162. Fig. 12A exemplifies the situation when the prosthesis is donned or connected and in the proper position (despite pistoning, rotation, volume changes, etc.). Fig. 12B exemplifies when the prosthesis is doffed or disconnected, such that there is a misalignment of the electrodes of the socket 14 to the conductive pads 12 of the liner.

It follows that the donning and doffing system can use with the EMG liner solution such that the user dons the EMG liner by rolling it onto the residual limb and then steps into the socket with embedded dome electrodes. When the electronic board determines the socket is properly donned, the EMG recording system will measure the muscle activity within the liner through the textile electrodes during daily activities and send EMG activity to the relevant prosthesis. When the electronic board determines the socket is not properly donned, the EMG recording system indicates to the user and prosthesis that the signal is unreliable and stops sending EMG activity to the prosthesis. If the doffed situation persists, the EMG recording system will stop measuring the muscle activity and hibernate.

The textile on the liner can all be conductive and make the conductive pads irrelevant, but user needs to completely doff the socket for the electronic board to determine the doffed situation. A socket fit indicator can be provided to the CPO/user via an App or LEDs. The conductive elements in the socket are pressure sensors instead and can determine if the user is loading the prosthesis or not. The conductive elements in the socket can be Hall-effect sensors instead and can measure magnetic field from embedded magnets located in the liner.

Fig. 11C exemplifies an alternative solution, whereby the skin S of the user can be used as the conductor between the electrodes or pogo pins 158, 160 of the socket 14 and connected to the electronics 162, although higher resistance can be expected. As a result, the user's skin can be used as the conductive pad when no liner is used.

Figs. 12A and 12B are associated with data obtained by a tracking system, including an EMG recording system with an IMU that measures EMG signals and inertial signals from the residual limb. The EMG Error Checker uses EMG values and the acceleration Jerk magnitudes to identify and remove motion artifacts in the EMG signals. The system enables the motion artifact to be reduced EMG control signal can lead to a stable EMG control for users. A usable surface EMG control signal is used for a prosthesis despite motion artifacts. Moreover, the system enables motion artifact removal without any additional sensors.

According to a method associated with the system, a user dons the EMG liner by rolling it onto the residual limb and then stepping into the socket with embedded dome electrodes. The EMG recording system measures the muscle activity within the liner through the textile electrodes during daily activities and sends EMG activity to the relevant prosthesis. Before sending the EMG activity to the prosthesis, the EMG control signal is calculated.

Calibration of the EMGJerk threshold may be necessary to tailor the threshold to each user. That is done by calculating the EMGJerk when the user contracts the muscles while stationary. The maximum calculated value, or any value above, is then set as the EMGJerk threshold so voluntary contractions will not be affected by the method.

According to the method, EMG signals are measured (1000Hz) from one or more muscles from the residual limb. Subsequently, high-pass (75Hz) and low-pass (400Hz) Butterworth filtering of the EMG signals occurs. The change in EMG is calculated for each channel deltaEMG(i) = EMG(i)-EMG(i-1). The change in acceleration is calculated for each axis deltaAcc(i) = Acc(i)-Acc(i-1) to get the Jerk. The magnitude of Jerk is calculated for 1 or more (X,Y,Z) axis Jerk(i) =sqrt(deltaAccX²+ deltaAccY²+ deltaAccZ²). The EMGJerk value for each EMG channel is calculated such that EMGJerk = abs(EMG(i) * deltaEMG(i) ) * Jerk(i). The EMGJerk is compared to a set threshold to determine if there is a motion artifact and if true, set EMG(i) to zero. A low-pass (2 Hz) Butterworth filter is applied to the signal to get the EMG Control Signal.

Fig. 12A shows data of level Ground walking with two voluntary contractions during the swing phase, signal from one muscle. An outer periphery of the plot demonstrates the raw EMG signal (defined as RAW), and the area of the plot below the raw EMG signal is the filtered signal (both low- and high-pass filtering) (defined as FILTERED). A voluntary contraction is designated at 170, the motion artifact is designated at 172, and the voluntary contraction while ambulating on level ground is designated at 174.

Fig. 12B shows that the control signal becomes more visible and useable after reducing motion artifacts. The EMG control signal (low-pass filtered) (defined as Controlx10), shown as a single line, is located above the motion artifact reduced EMG signal (defined as FILTERED+MotionArtifactReduced). Voluntary contraction is designated at 176, the motion artifact is designated at 178, and the voluntary contraction while ambulating on level ground is designated at 180.

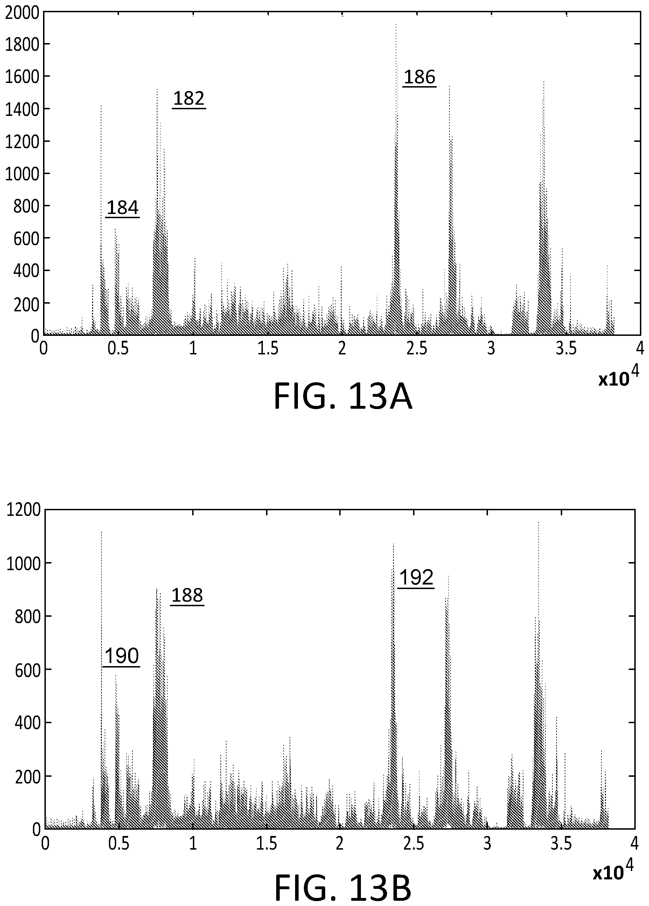

According to Figs. 13A and 13B, is another artifact removal system. Similarly to the above, a user dons the EMG liner by rolling it onto the residual limb and then stepping into the socket with embedded dome electrodes. The EMG recording system measures the muscle activity within the liner through the textile electrodes during daily activities and send EMG activity to the relevant prosthesis. Before sending the EMG activity to the prosthesis, the EMG control signal is calculated.

According to the method, EMG signals are measured (1000Hz) from one or more muscles from the residual limb. Subsequently, high-pass (75Hz) and low-pass (400Hz) Butterworth filtering of the EMG signals occurs. The average EMG value is calculated, preferably for the previous 10 samples and for the previous 90 samples prior to the 10 samples, for each EMG channel avgOld(i) = ∑EMG(i-100:i-10)/90, avgNew(i) =∑EMG(i-9:i)/10, where i is the current EMG sample. If the average of the recent 10 samples are significantly larger than the average of the 90 samples prior then it is likely the measured sample is an artifact that we want to remove. avgNew > avgOld*1.7 then the current EMG sample is set to the average of the 90 samples EMG(i) = avgOld(i) to reduce the effect of the artifact. A low-pass (2 Hz) Butterworth filter is applied to the signal to get the EMG Control Signal.

Fig. 13A represents a level ground walking with two voluntary contractions during swing phase, signal from one muscle, with plots associated with the foregoing system and method. The top portion of the plot represents a raw EMG signal, whereas the underlying portion of the plot represents a filtered EMG signal (low- and high-pass filtering). Voluntary contraction is designated at 182, the motion artifact is designated at 184, and the voluntary contraction while ambulating on level ground is designated at 186.

Fig. 13B represents the top portion of the plot with the filtered EMG signal (low- and high-pass filtering), the portion below the top portion is the motion artifact reduced EMG signal, whereas a traceable line within the foregoing plot represents the EMG control signal with low-pass filtering. A voluntary contraction is designated at 188, the motion artifact is designated at 190, and the voluntary contraction while ambulating on level ground is designated at 192.

Alternative solutions may be provided according to the system and method. EMG may be measured with higher or lower frequencies from one or more channels. Accelerations may measure with higher or lower frequencies with low and/or high-pass filtering. EMG filtering may occur at other frequencies. EMG filtering may use other method of filtering. Jerk can be calculated from 1-3 axes of acceleration. Either EMG values or change in EMG values can be omitted from the equation. Either acceleration values or changes in acceleration values can be omitted from the equation. The change in angular velocity (measured by a gyroscope) can also be used instead of the acceleration Jerk. The final low-pass filtering to get the EMG control signal can also be done by performing other filtering methods and/or calculating the signal's envelope, mean average, etc. Using another sensor on or inside the socket can indicate motion artifacts, e.g., Hall-effect sensor on the socket with a magnet inside the liner, force sensor, strain-gauges. The prosthesis can provide information about gait phases to indicate motion artifacts, as the motion artifacts happen mostly around heel-strike and toe-off.

It is to be understood that not necessarily all objects or advantages may be achieved under any embodiment of the disclosure. Those skilled in the art will recognize that the prosthetic assembly and electrode interface may be embodied or carried out in a manner that achieves or optimizes one advantage or group of advantages as taught herein without necessarily achieving other objects or advantages taught or suggested herein.

The skilled artisan will recognize the interchangeability of various disclosed features. The skilled artisan will understand that the features described herein may be adapted to other types of devices. Hence this disclosure and the embodiments and variations thereof are not limited to liners for prosthetic devices but can be utilized in any device.

Although this disclosure describes certain exemplary embodiments and examples of a liner, it will be understood by those skilled in the art that the present disclosure extends beyond the specifically disclosed embodiments to other alternative embodiments and/or uses of the disclosure and obvious modifications thereof. It is intended that the present disclosure should not be limited by the disclosed embodiments described above and may be extended to other applications that may employ the features described herein.

## Claims

1. An electrode interface (20, 50, 60, 70) for a prosthetic assembly (10) comprising a prosthetic liner (12) and a prosthetic socket (14), the electrode interface comprising: an electrode configured to be secured to the liner (12, 122) with an electrical conduit arranged to transfer an electrical potential from an interior side (96, 126) of the prosthetic liner to an exterior side (98, 128) of the prosthetic liner,
wherein the electrode defines an inner portion (34) configured to cover a predetermined surface area (w2) of the interior side of the liner, an outer portion (36) and a connecting portion (38, 40) connecting the inner portion to the outer portion,
wherein the interior side of the prosthetic liner has a body portion defined by an elastomeric material and the electrical conduit is configured to extend from an inner portion of the electrode on the interior side of the prosthetic liner to the exterior side of the body portion along which extends a textile layer (94, 124) of the liner (102),
wherein the inner portion, the outer portion and the connecting portion are defined by a conductive textile (58),
**characterized in that** the outer portion (36) is configured to cover a predetermined surface area of the exterior side of the liner, the connecting portion (38, 40) is sized and configured to a width of the prosthetic liner (12, 122), wherein the outer portion (36) of the electrode is configured to extend along the exterior side of the body portion and to be flush with said textile layer (94, 124) of the liner (12, 122), and be exposed along the textile layer (94, 124).

2. The electrode interface of claim 1, further comprising a dome electrode (42) configured to be connected to the socket (14) and configured to connect to the electrode to provide for electrical contact.

3. The electrode interface of claim 2, wherein the dome electrode (42) is configured to relay signals to an amplifier.

4. The electrode interface of claim 3, wherein the outer portion of the electrode is configured to be larger than the dome electrode.

5. The electrode interface of claim 1, wherein the electrical conduit is configured to be embedded in a thickness of the prosthetic liner extending between the interior and exterior sides of the prosthetic liner.

6. The electrode interface of claim 1, further comprising at least a further electrode configured to be included in a prosthetic liner extending along the exterior side of the prosthetic liner.

7. The electrode interface of claim 1, wherein the outer portion includes first and second outer portions (32, 36) spaced apart by the inner portion (34), wherein first and second connecting portions (38, 40) connect the first and second outer portions to the inner portion, respectively.

8. The electrode interface of claim 7, wherein the first outer portion (36) defines a shape and the second outer portion (32) defines a shape having less surface area than the first outer portion.

9. The electrode interface of claim 1, wherein the outer portion (36) defines a circular shape.

10. The electrode interface of claim 1, wherein the inner portion (34) defines an elongated shape.

11. A method for using an electrode interface in a prosthetic assembly including a prosthetic liner and a prosthetic socket, the method comprising the steps of: including the electrode interface of claim 1 in a prosthetic liner (12, 122); donning the prosthetic liner onto a residual limb; corresponding the electrode interface with an dome electrode on the prosthetic socket; recording signals from the electrode interface via the dome electrode to measure muscle activity of the residual limb.

## Patentansprüche

1. Eine Elektrodenschnittstelle (20, 50, 60, 70) für eine Prothesenanordnung (10), umfassend eine Prothesenauskleidung (12) und eine Prothesenbuchse (14), wobei die Elektrodenschnittstelle umfasst: eine Elektrode, die konfiguriert ist, um an der Auskleidung (12, 122) gesichert zu werden, mit einer elektrischen Leitung, die angeordnet ist, um ein elektrisches Potenzial von einer Innenseite (96, 126) der Prothesenauskleidung zu einer Außenseite (98, 128) der Prothesenauskleidung zu übertragen,
wobei die Elektrode einen Innenabschnitt (34), der konfiguriert ist, um einen vorbestimmten Oberflächenbereich (w2) der Innenseite der Auskleidung zu bedecken, einen Außenabschnitt (36) und einen Verbindungsabschnitt (38, 40) definiert, der den Innenabschnitt mit dem Außenabschnitt verbindet,
wobei die Innenseite der Prothesenauskleidung einen Körperabschnitt aufweist, der durch ein Elastomermaterial definiert wird, und die elektrische Leitung konfiguriert ist, um sich von einem Innenabschnitt der Elektrode an der Innenseite der Prothesenauskleidung zur Außenseite des Körperabschnitts zu erstrecken, dem entlang sich eine Textilschicht (94, 124) oder Auskleidung (102) erstreckt,
wobei der Innenabschnitt, der Außenabschnitt und der Verbindungsabschnitt durch ein leitfähiges Textil (58) definiert sind,
**dadurch gekennzeichnet, dass** der Außenabschnitt (36) konfiguriert ist, um einen vorbestimmten Oberflächenbereich der Außenseite der Auskleidung zu bedecken, der Verbindungsabschnitt (38, 40) auf eine Breite der Prothesenauskleidung (12, 122) bemessen und konfiguriert ist, wobei der Außenabschnitt (36) der Elektrode konfiguriert ist, um sich entlang der Außenseite des Körperabschnitts zu erstrecken, und mit der Textilschicht (94, 124) der Auskleidung (12, 122) bündig zu sein, und entlang der Textilschicht (94, 124) freigelegt ist.

2. Die Elektrodenschnittstelle nach Anspruch 1, weiter eine Kuppel-Elektrode (42) umfassend, die konfiguriert ist, um mit der Buchse verbunden zu werden (14) und konfiguriert ist, um die Elektrode zu verbinden, um einen elektrischen Kontakt bereitzustellen.

3. Die Elektrodenschnittstelle nach Anspruch 2, wobei die Kuppel-Elektrode (42) konfiguriert ist, um Signale an einen Verstärker weiterzuleiten.

4. Die Elektrodenschnittstelle nach Anspruch 3, wobei der Außenabschnitt der Elektrode konfiguriert ist, um größer als die Kuppel-Elektrode zu sein.

5. Die Elektrodenschnittstelle nach Anspruch 1, wobei die elektrische Leitung konfiguriert ist, um in eine Dicke der Prothesenauskleidung eingebettet zu sein, die sich zwischen der Innen- und Außenseite der Prothesenauskleidung erstreckt.

6. Die Elektrodenschnittstelle nach Anspruch 1, weiter mindestens eine weitere Elektrode umfassend, die konfiguriert ist, um in einer Prothesenauskleidung beinhaltet zu sein, die sich entlang der Außenseite der Prothesenauskleidung erstreckt.

7. Die Elektrodenschnittstelle nach Anspruch 1, wobei der Außenabschnitt erste und zweite Außenabschnitte (32, 36) beinhaltet, die von dem Innenabschnitt (34) beabstandet sind, wobei der erste und zweite Verbindungsabschnitt (38, 40) jeweils den ersten und zweiten Außenabschnitt mit dem Innenabschnitt verbinden.

8. Die Elektrodenschnittstelle nach Anspruch 7, wobei der erste Außenabschnitt (36) eine Form definiert und der zweite Außenabschnitt (32) eine Form definiert, die einen kleineren Oberflächenbereich als der erste Außenabschnitt aufweist.

9. Die Elektrodenschnittstelle nach Anspruch 1, wobei der Außenabschnitt (36) eine Kreisform definiert.

10. Die Elektrodenschnittstelle nach Anspruch 1, wobei der Innenabschnitt (34) eine längliche Form definiert.

11. Ein Verfahren zum Verwenden einer Elektrodenschnittstelle in einer Prothesenanordnung, die eine Prothesenauskleidung und eine Prothesenbuchse beinhaltet, wobei das Verfahren die Schritte umfasst zum:
Beinhalten der Elektrodenschnittstelle nach Anspruch 1 in einer Prothesenauskleidung (12, 122);
Anlegen der Prothesenauskleidung an einem Stumpf; Übereinstimmen der Elektrodenschnittstelle mit einer Kuppel-Elektrode an der Prothesenbuchse; Aufzeichnen von Signalen von der Elektrodenschnittstelle über die Kuppel-Elektrode, um Muskelaktivität des Stumpfes zu messen.

## Revendications

1. Une interface d'électrode (20, 50, 60, 70) pour un ensemble prothétique (10) comprenant une doublure prothétique (12) et une emboîture prothétique (14), ladite interface d'électrode comprenant : une électrode configurée pour être fixée à la doublure (12, 122) avec un conduit électrique agencé pour transférer un potentiel électrique d'un côté intérieur (96, 126) de la doublure prothétique à un côté extérieur (98, 128) de ladite doublure prothétique, dans lequel l'électrode définit une partie intérieure (34) agencée pour couvrir une surface prédéterminée (w2) du côté intérieur de la doublure, une partie extérieure (36), et une partie de connexion (38, 40) reliant la partie intérieure à la partie extérieure, dans lequel le côté intérieur de la doublure prothétique a une partie corporelle définie par un matériau élastomère et le conduit électrique configuré pour s'étendre d'une partie intérieure de l'électrode sur le côté intérieur de la doublure prothétique au côté extérieur de la partie corporelle le long de laquelle s'étend une couche textile (94, 124) de la doublure (102),
dans lequel la partie intérieure, la partie extérieure et la partie de connexion sont définies par un textile conducteur (58),
**caractérisé en ce que** la partie extérieure (36) est configurée pour couvrir une surface prédéterminée du côté extérieur de la doublure, la partie de connexion (38, 40) est dimensionnée et configurée à une largeur de la doublure prothétique (12, 122), dans lequel la partie extérieure (36) de l'électrode est configurée pour s'étendre le long du côté extérieur de la partie corporelle et pour être à niveau avec ladite couche textile (94, 124) de la doublure (12, 122), et être exposée le long de la couche textile (94, 124).

2. L'interface d'électrode de la revendication 1, comprenant en outre une électrode en dôme (42) configurée pour être connectée à l'emboîture (14) et configurée pour se connecter à l'électrode afin de fournir un contact électrique.

3. L'interface d'électrode de la revendication 2, dans laquelle l'électrode en dôme (42) est configurée pour relayer des signaux à un amplificateur.

4. L'interface d'électrode de la revendication 3, dans laquelle la partie extérieure de l'électrode est configurée pour être plus grande que l'électrode en dôme.

5. L'interface d'électrode de la revendication 1, dans laquelle le conduit électrique est configuré pour être intégré dans une épaisseur de la doublure prothétique s'étendant entre les côtés intérieur et extérieur de ladite doublure prothétique.

6. L'interface d'électrode de la revendication 1, comprenant en outre au moins une autre électrode configurée pour être incluse dans une doublure prothétique s'étendant le long du côté extérieur de la doublure prothétique.

7. L'interface d'électrode de la revendication 1, dans laquelle la partie extérieure comprend des première et seconde parties extérieures (32, 36) espacées par la partie intérieure (34), dans lequel des première et seconde parties de connexion (38, 40) relient respectivement les première et seconde parties extérieures à la partie intérieure.

8. L'interface d'électrode de la revendication 7, dans laquelle la première partie extérieure (36) définit une forme et la seconde partie extérieure (32) définit une forme ayant une surface inférieure à celle de la première partie extérieure.

9. L'interface d'électrode de la revendication 1, dans laquelle la partie extérieure (36) définit une forme circulaire.

10. L'interface d'électrode de la revendication 1, dans laquelle la partie intérieure (34) définit une forme allongée.

11. Un procédé d'utilisation d'une interface d'électrode dans un ensemble prothétique comprenant une doublure prothétique et une emboîture prothétique, ledit procédé comprenant les étapes de : inclure l'interface d'électrode de la revendication 1 dans une doublure prothétique (12, 122) ; enfiler la doublure prothétique sur un membre résiduel ; faire correspondre l'interface d'électrode avec une électrode en dôme sur l'emboîture prothétique ; enregistrer des signaux de l'interface d'électrode via l'électrode en dôme pour mesurer l'activité musculaire du membre résiduel.
